# EUROPEAN PATENT APPLICATION

(11) **EP 3 446 803 A1**
(43) Date of publication of application: **27.02.2019**
(21) Application number: 17187789.7
(22) Date of filing: 24.08.2017
(51) Int. Cl.: B21D 22/20

(54) **METHOD OF FORMING HOLLOW ARTICLES**

(71) Applicant: Presspart Manufacturing Ltd., Blackburn, Lancashire BB1 5RF (GB)
(72) Inventor: KRÄTZIG, Christian, 33181 Bad Wünnenberg (DE); WHALLEY, Mark, Langho, BB6 8DQ (GB); NOBLE, Alan William, Rossendale, BB4 4EE Lancashire (GB); TURNER, Richard, Simonstone Lancashire, BB12 7ST (GB); SEILER, Matthias, 40627 Düsseldorf (DE)
(74) Representative: Uexküll & Stolberg

(57) **Abstract**

A method of forming a hollow article (2) comprises the steps of providing a metal blank in the form of a disk. Subsequently, a metal blank is transferred to a first forming station which comprises a punch and a die. A metal blank is formed into a cup having a sidewall and a closed base. A boundary lubrication is provided between the die and the metal blank by application of a lubricant.

Hereinafter, the metal cup is provided to a set of forming stations in a deep drawing transfer press. The set of forming stations comprises at least one set of punches and dies. Said metal cup is deep drawn into a hollow article (2) having a sidewall (4) and a closed base (7) with an inner and an outer surface (, 8, 6, 9) by the action of said at least one set of punches and dies. Subsequently, the hollow article (2) is provided to a subsequent forming station in the deep drawing transfer press.

Hereinafter, an individual identification mark is supplied to the outer surface (6, 9) of the container sidewall (4) and/or of the base (7) by means of laser engraving at said subsequent forming station.

## Description

### FIELD OF THE INVENTION

The present invention relates to a method of forming hollow articles of sheet material, particularly metal, by deep drawing with the help of at least one punch having projecting forming surfaces and of means stretching the sheet material across the punch while the latter is moved traversly to the material, in particular at least one die which receives the formed article. Such deep drawn hollow articles, particularly cylindrical articles find application in very different fields such as containers for medicaments in metered dose inhalers (MDI), as battery cups or as cartridge casings for firearm ammunition. The term cylindrical is not meant to be restricted to a circular cylinder, although this form might be a preferred one. The term cylindrical may also include cylinders having a polygonal or elliptical base.

### BACKGROUND OF THE INVENTION

Hollow metal articles, such as cylindrical metal containers are typically deep drawn from metal cups, which are formed from metal sheets, often also referred-to as metal blanks. As defined in industry norm DIN 8584, the deep drawing process is a forming process which occurs under a combination of tensile and compressive conditions. A flat sheet metal blank is formed into a hollow body open on at least one side or a hollow body is formed into a hollow body with a smaller cross-section.

A typical deep drawing process is e.g. described in US Patent 2 989 019, which is herewith incorporated by reference.

In forming hollow metal articles by deep drawing it is customary to tightly hold opposite marginal regions or the entire circumferential region of a sheet metal blank and to exert a force restraining the marginal regions from moving toward the center of the punch during the forming operation. Typically, the metal sheet or the metal cup is drawn to the desired length by a number of forming operations involving a number of punches and dies using a transfer press. After the drawing process the formed article is discarded from the machine, such as into a collecting tray or collecting vessel.

During the deep drawing the material undergoes high stress loads. Characteristic of deep drawing is the high pressure on the order of 100,000 pounds per square inch (psi) involved in the operation. To deal with such force, a lubricant is used and the choice of lubricant is critical to the success of the operation. Under such pressure, the drawing lubricant should cool the die and the article, provide boundary lubrication between the die and the article, prevent metal-to-metal adhesion or welding, and cushion the die during the drawing operation.

Nevertheless known procedures and equipment lead to conditions in which the metal blank is subjected to different stretching in different areas so that the finished article has varying thickness. Often the different stretching leads to rupturing the blank while in other regions the stretch is so small that practically no change of the original thickness of the blank is obtained.

Ruptures or other failures may not be visible straightaway but may become perceivable during later processing, e.g. during filling with matter such as a medicament or during the shelf life of the finished article. In such a case it would be desirable to identify the batch of articles the failed article belongs to in order to check the entire batch. In case of metered dose inhalers certain regulatory entities require information about source and fabricator, item number, composition and quality number, etc. of the container containing the medication for approval of the medication. It would be desirable to make this information accessible to the drug manufacturer directly, where the medicament container, in particular the MDI container is typically filled and labeled.

Labels on an article also allow the provision of additional information for later productions steps and/or the end customer such as for example the date of production, the filling, the material or the inner coating of the hollow article, where applicable. However, so far labeling of a hollow article occurs only after it has been filled. In case a sticker is used as a label washing of the hollow article before labeling is required in order to remove the lubricant and allow proper adherence of the sticker. Labeling a hollow article after it was filled is disadvantageous as the allocation of the labeled hollow article to the corresponding batch of hollow articles according to their order of production is no longer possible.

### DESCRIPTION OF THE INVENTION

Therefore, it is an object of the present invention to provide a method of forming hollow articles with a label, wherein the label provides as comprehensive information of the article as possible, in particular wherein each article can be identified according to its order of production by use of the label.

In connection with the present invention it has been found that the most suitable time to label the hollow article is during its production, in particular during or subsequent to its forming. However, as the article is coated with a lubricant during the deep drawing operations labels in form of stickers will not adhere to the lubricated and inks cannot be suitably applied.

Therefore it is another object of the invention to provide a method of forming a hollow article by which a labeled hollow article may be fabricated, wherein the label does not only provide as comprehensive information of the article as possible but also wherein the label may be applied to the article despite of a lubricant the article is coated with.

Accordingly, the present invention relates to a method of forming a hollow article comprising the steps of claim 1 as well as to a medicament container according to claim 11. Preferred embodiments of the method and the hollow article are set out in the respective dependent claims.

According to the present invention, an individual identification mark is applied to the outer surface of the article sidewalls and/or of the base by means of laser engraving subsequent to the deep drawing of the cup into the hollow article in the deep drawing transfer press. Alternatively, the identification mark is applied to the outer surface of the article sidewalls and/or of the base by means of laser engraving subsequent to the deep drawing transfer press. Preferably the identification mark is applied to the article directly on leaving the press, before bulk collection or between the deep drawing transfer press and washing of the article.

According to the present invention, each article is engraved in an order corresponding to which the articles exit the deep drawing transfer press which, in the following, is referred to as the order of production. Identification of each hollow article in its order of production ensures that a batch to which one single hollow article belongs can be identified. For example, if one hollow article has a production fault, the additional articles which were produced directly prior or subsequent to the damaged article may also be identified, checked for damages or rejected.

In the following the terms "label" and "identification mark" will be used as synonyms and are thus interchangeable.

Each identification mark is individually assigned to each of the hollow articles. Each identification mark is unique such that each hollow article may be individually recognized by its identification mark. The identification mark may be a 2D-code such as a QR-code or a data matrix, a bar code, a numerical code, an alphanumerical code or any other suitable identification means. Preferably the identification mark is scannable by a mobile device such as a smartphone.

Preferably the hollow article is formed of metal, more preferably of steel, aluminum or brass. A person skilled in the art readily understands that also other metals may be used for drawing hollow articles from metal cups or blanks under use of a deep drawing transfer press.

In a preferred embodiment of the present invention, the cup has the form of a hollow cylinder. Preferably the cup is formed of a metal disk at the same deep drawing transfer press in which all other forming operations take place.

According to another preferred embodiment, the deep drawing transfer press is a multiple station deep drawing transfer press. Multiple station deep drawing transfer presses allow the integration of multiple deep drawing forming operations in one single press. In particular, the step of applying an individual identification mark to the outer surface of the sidewall and/or the outer surface of the base of the hollow article is performed within the same multiple station deep drawing transfer press.

Preferably, there are no intermediate washing steps between steps (a) and (d) as defined in claim 1. According to step (a) the metal blank is provided. According to step (d) the identification mark is applied. Washing of the hollow article may take place after step (d). The step of applying the individual identification mark is performed with the hollow article being coated by a lubricant. In connection with the present invention it has been found that the quality of the laser engraved identification mark is not essentially influenced by a lubricant provided that the laser radiation is focused on the outer surface of the hollow article which is to be engraved. Thus, the same quality of a laser engraved identification mark applied to a non lubricated hollow article could be maintained in connection with the present invention when laser engraving a lubricated hollow article.

In a further embodiment, there are further forming steps applied to the hollow article after step (c) or step (d) depending on whether the identification mark is applied to the article in or subsequent to the deep drawing transfer press. In case the identification mark is applied in the deep drawing transfer press further forming steps may be performed after step (d). In case the identification mark is applied subsequent to the deep drawing transfer press the further forming steps may be performed after step (c). Subsequent forming steps may be directed to further forming of the base, for example the base may be formed into a dome shape, or to an end of the hollow article opposing the base. The opposing end may be provided with a neck portion or may undergo additional forming steps. In case a dome shaped base of the hollow article is intended the identification mark is optionally engraved in the flat base of the hollow article prior to the forming of the dome shape. Alternatively the identification mark may also be engraved in the dome-shaped base. Preferably the hollow article has a hollow cylindrical shape.

Also preferred is an embodiment according to which the identification mark is a 2D-code, in particular a QR-code or a data matrix. 2D-codse are very common. There are apps for mobile devices which allow reading of 2D-codes. Consequently 2D-codes will allow end customers to access information associated with such an identification mark in a cost effective manner.

In a preferred embodiment, the hollow article is a metal container, a battery cup, an aerosol container, a cartridge casing, a sink or a pot. Thus, the hollow article is preferably configured to receive the battery components such as the anode and cathode or is configured to receive medicaments under pressure in a liquid form. Moreover, the hollow article may take the form of any hollow article which may be fabricated by deep drawing operations as described above.

It is also preferred that the lubricant is selected from the group consisting of talcum, mineral oil, graphite, molybdenum disulphide, heavy duty emulsions, phosphates, white lead and wax films. Lubricants are used in general to reduce friction between the working material and the punch and die, cool the parts during deep drawing and cleaning the deep drawing tools. They also aid in removing the part from the punch and thus play an important role in the deep drawing process as they enhance the probability to produce a good quality part.

In another preferred embodiment at least one database is provided in order to store data related to each hollow article, wherein the individual identification mark is associated with the data of each hollow article. For example, any data relating to the hollow article itself as well as to its production may be collected and stored in such a database. Such data may be for example the date and location of production, the intended use, the content of the hollow article, the size and/or the manufacturer, the location of assembly with other components and/or the location where the hollow article is marketed. Optionally additional data may be added to the database during a life circle of the hollow article. For example when the hollow article is a metal container which is to be assembled to a medicament container at a different plant than where the metal container was deep drawn, arrival of the metal container at the assembly location may be stored in the database. When several locations of the hollow article during its life circle are known the hollow article is traceable at least to a certain degree. Optionally the location of each scan of the identification mark may be saved to a database. Moreover, the identification mark may be scannable in order to proof the authentication of the hollow article. For example when scanning the identification mark a request may be sent to a database requesting proof of the authenticity of the hollow article. If the identification mark and the associated hollow article are for example known by the database a message confirming the authenticity is send in return. Optionally, there are multiple databases each containing a different set of data associated with the hollow article.

Preferably, an interface for accessing at least one database is provided, wherein the database is accessible by an end customer. The identification mark may comprise a link which allows access of the associated data contained in the at least one database and/or enables the user to verify the authenticity of the hollow article. The identification mark may link a user depending on its permission to access data of different security levels to different databases. For example, an end customer might only be allowed to access data relating to the date and location of production of the hollow article whereas an employee of the manufacturer of the hollow article being involved in the development of such a hollow article may be allowed to access detailed data related to, e.g. detailed fabrication information. This access may be association with entering a password and/or the provision of other suitable proofs of identity.

Optionally an end customer may scan the identification mark using a smartphone having a dedicated application (app) installed thereon. In connection with the present invention problems have been encountered with regard to smartphones focusing on the rim of the article instead on the curved base when trying to scan the identification mark on the base of the article. According to the present invention the app might be specifically designed for the reading of the identification mark. In particular the app might be adapted to control and offset the focus of the smartphone in order to allow precise scanning of the identification mark on the curved base of the article. For example the app might use curve compensation for the shape of the base of the article in order to further improve readability of the identification mark via the smartphone. Moreover, correction for reflection and light level of the curved surface of the article might be implemented in the dedicated app. Further, such a dedicated app would not only improve readability of the identification mark but would also allow some control of how the phone uses the information of the identification mark and the information the customer is provided with accessing the database via the dedicated app.

According to another embodiment, the present invention relates to a medicament container comprising a hollow article in the form of a metal container, in particular an aerosol container, and a top cover, wherein the metal container is formable by the method described above. Preferably such medicament container is formed as an MDI container, which is configured to be inserted into a metered dose inhaler. Such metered dose inhalers allow inhalation of doses of medicament. The MDI container may be replaced by another container when being empty.

### DETAILED DESCRIPTION OF THE INVENTION

In the following, a preferred embodiment of the invention is explained in detail with reference to the drawings. A person skilled in the art will readily understand that this embodiment is only an exemplary realization of the invention and is not to be understood as limiting the claimed scope. In the drawings:
- Figure 1: shows a perspective view of a medicament container and
- Figure 2: shows a cross-sectional view of a hollow article in form of an aerosol container.

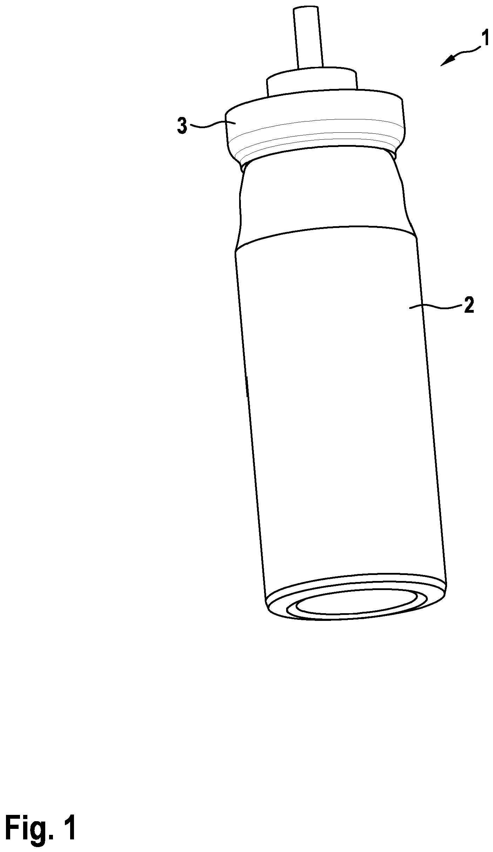

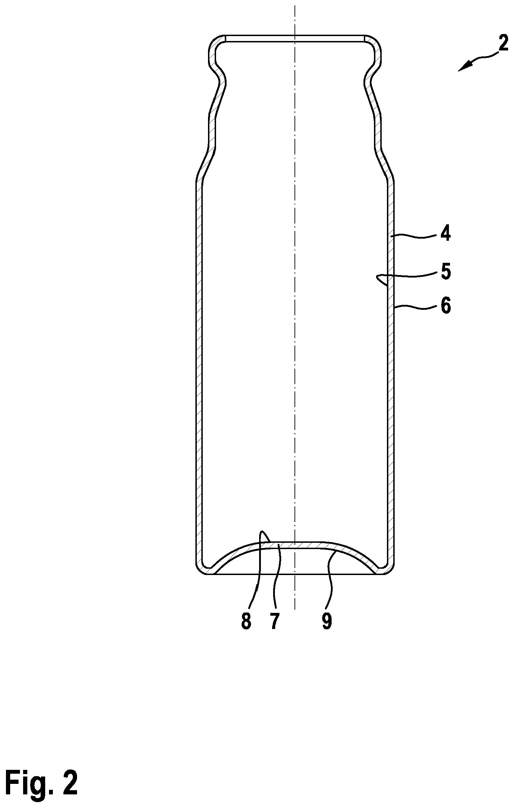

As disclosed in Figures 1 and 2, a medicament container in the form of a MDI container 1 comprises a hollow article formed as a metal container, in particular formed as an aerosol container 2, and a top cover 3.

The aerosol container 2 has a sidewall 4 with an inner surface 5 and an outer surface 6. The aerosol container 2 further comprises a closed base 7 which has a dome shape and comprises an inner surface 8 and an outer surface 9. An identification mark in form of a 2D-code, in particular a data matrix or a QR-code (not shown) is engraved on the outer surface 9 of the base 7 by use of laser radiation. The 2D-code allows identification of each aerosol container 2 in its order of production.

A person skilled in the art will readily understand that the invention is not limited to a medicament container or to a metal container, respectively. For example the hollow article may also be formed as a battery cup used for the production of a battery or formed as a cartridge casing for firearm ammunition. Moreover, it has to be understood that the identification mark may also be formed as a bar code, a numerical or alphanumerical code or any other suitable identification means. The identification mark may be engraved on the outer surfaces 6, 9 of the sidewall 4 and/or of the base 7.

In the following, a method of forming a hollow article shall be explained in connection with Figures 1 and 2 by means of the forming of a metal container, in particular an aerosol container 2:
In a first step, a metal blank in the form of a disk is provided. The metal blank may be a purchased part. However, it is intended to fabricate the metal blank of a metal sheet by die cutting using the same deep drawing transfer press which is a multiple station deep drawing transfer press by means of which all other deep drawing operations according to the present invention are performed.

Subsequently, said metal blank is transferred to a first forming station which comprises a punch and a die, wherein the metal blank is formed into a cup which has the form of a hollow cylinder. The cup has a sidewall and a closed base. The cup may also be a purchased part. However, as explained above with regard to the metal blank it is intended to form the cup of the metal blank using the same multiple station deep drawing transfer press by means of which all other deep drawing operations according to the present invention are performed.

Prior to the first forming station, boundary lubrication is provided between the die and the metal blank by application of a lubricant. The lubricant is chosen out of the group of the following lubricants: talcum, mineral oil, graphite, molybdenum disulphide, heavy duty emulsions, phosphates, white lead and wax films.

Subsequently, the metal cup is provided to a set of forming stations in the multiple stations deep drawing transfer press. The set of forming stations comprises at least one set of punches and dies. Said metal cup is deep drawn into hollow article formed as a metal container, in particular formed as an aerosol container 2, by the action of said at least one set of punches and dies. As described above the aerosol container 2 has a sidewall 4 and a closed base 7 with an inner surface 5, 8 and an outer surface 6, 9.

Thereafter, the aerosol container 2 is provided to a subsequent forming station in the multiple station deep drawing transfer press. Subsequently, an individual identification mark in form of a 2D-code, in particular a data matrix or a QR-code is applied to the outer surface 9 of the base 7 by means of laser engraving at said subsequent forming station. However, there is no need to say that the 2D-code may also be applied to the outer surface 6 of the canister sidewall 4.

Hereinafter, there may be further forming steps applied to the aerosol container 2. These forming steps may contain the forming of the aerosol container 2 at an end located opposite to the base 7. Moreover, these further forming steps may contain the forming of the base 7 into a dome shape. Alternatively, the dome shape of the base 7 is formed prior to the step of laser engraving the identification mark, in particular when the metal cup is deep drawn into an aerosol container 2 by the action of said at least one set of punches and dies.

However, it is also possible to apply the individual identification mark in form of a 2D-code by means of laser engraving subsequent to the deep drawing transfer press instead, wherein each article is engraved in an order corresponding to which the articles exit the deep drawing transfer press. Preferably the identification mark is applied to the article directly on leaving the press, before bulk collection or between the deep drawing transfer press and washing of the article.

In between the steps of providing a metal blank in the form of a disk (step d) and the laser engraving of an identification mark (step e), there are no intermediate washing steps. Consequently, the aerosol container 2 remains covered with a lubricant while the individual identification mark is laser engraved. Washing of the aerosol container 2 may take place hereinafter.

Preferably prior to providing the metal blank in the form of a disk, at least one database is provided in order to store data related to each aerosol container 2, wherein the 2D-code is associated with the data of each aerosol container 2. Additionally, an interface for accessing at least one database is provided, wherein the database is accessible by an end customer.

Multiple databases may be provided, wherein different sets of data are stored in the different databases. Moreover, the 2D-code may be configured to link the person which is, for example, scanning the 2D-code, to different data bases depending on the security level of the scanning person. For example, an end customer may be authorized to access a different database containing different data compared to an engineer being involved in the development of aerosol containers. Optionally the end customer might use a smartphone having a dedicated app installed thereon in order to scan the 2D-code.

## Claims

1. A Method of forming multiple hollow articles (2) one after another comprising the steps of:
(a) providing a metal blank in the form of a disk;
(b) transferring said metal blank to a first forming station which comprises a punch and a die, wherein the metal blank is formed into a cup having sidewalls and a closed base and, wherein boundary lubrication is provided between the die and the metal blank by application of a lubricant;
(c) providing the metal cup to a set of forming stations in a deep drawing transfer press, the set of forming stations comprising at least one set of punches and dies, wherein said metal cup is deep drawn into a hollow article (2) having a sidewall (4) and a closed base (7) with an inner and an outer surface (5, 8, 6, 9) by the action of said at least one set of punches and dies;
**characterized by**
(d) applying an individual identification mark to the outer surface (6, 9) of the article sidewall (4) and/or of the base (7) by means of laser engraving at a subsequent forming station in the deep drawing transfer press or subsequent to the deep drawing transfer press, wherein each article is engraved in an order corresponding to which the articles exit the deep drawing transfer press.

2. Method according to claim 1 **characterized in that** the cup has the form of a hollow cylinder.

3. Method according to any of the preceding claims, **characterized in that** the deep drawing transfer press is a multiple station deep drawing transfer press.

4. Method according to any of the preceding claims, **characterized in that** between steps (a) and (d), there are no intermediate washing steps.

5. Method according to any of the preceding claims, **characterized in that** after step (c) or step (d) there are further forming steps applied to the hollow article (2).

6. Method according to any of the preceding claims, **characterized in that** that the identification mark is a 2D-code, in particular a QR-code or a data matrix.

7. Method according to any of the preceding claims, **characterized in that** the hollow article (2) is a metal container, a battery cup, an aerosol container (2), a cartridge casing, a sink or a pot.

8. Method according to any of the preceding claims, **characterized in that** the lubricant is chosen out of the group of the following lubricants: talcum, mineral oil, graphite, molybdenum disulphide, heavy duty emulsions, phosphates, white lead and wax films.

9. Method according to any of the preceding claims, **characterized by** the step of providing at least one database in order to store data related to each hollow article (2), wherein the individual identification mark is associated with the data of each hollow article (2).

10. Method according to claim 10, **characterized by** the step of providing an interface for accessing at least one database, wherein the database is accessible by an end customer.

11. Medicament container comprising a hollow article in the form of a metal container (2) and a top cover (3), wherein the metal container (2) is formable by a method according to any of the preceding claims.

12. Medicament container according to claim 12 **characterized in that** the medicament container (1) is formed as an MDI container.

13. Medicament container according to claims 12 or 13 **characterized in that** the medicament container (1) is formed as an MDI container which is configured to be inserted into a metered dose inhaler.
